# EUROPEAN PATENT APPLICATION

(11) **EP 2 322 189 A2**
(43) Date of publication of application: **18.05.2011**
(21) Application number: 10177819.9
(22) Date of filing: 15.02.2005
(51) Int. Cl.: A61K 31/727, A61K 38/36, A61K 38/55, A61P 11/00

(54) **Method of preventing fibrin clots in pulmonary tissue through the use of aerosolized anticoagulants**

(30) Priority: 20.02.2004 US 546236 P; 14.02.2005 US 57522
(62) Divisional of application: 05713495.9
(71) Applicant: Board of Regents, The University of Texas System, Austin, TX 78701 (US)
(72) Inventor: Enkhbaatar, Perenlei, Galveston, TX 77551 (US); Murakami, Kazunori, Galveston, TX 77551 (US); Traber, Daniel L., Galveston, TX 77551 (US)
(74) Representative: Williams, Gareth Owen

(57) **Abstract**

ATIII is a serine protease inhibitor (seipin) with anti-coagulant, anti-inflammatory, antiproliferative and anti-angiogenic properties. The invention features methods of treating a subject having lung injury due to burns and smoke inhalation by administering a synergistic combination of antithrombin III and heparin through pulmonary delivery means.

## Description

### PRIORITY CLAIM

This application claims priority to USSN 60/546,236, filed on February 20, 2004, the contents of which are incorporated herein by reference.

### BACKGROUND

In inflammatory lung diseases, fibrin is observed in the airways. Alveolar fibrin formation is known as a hallmark of acute and/or chronic inflammatory lung diseases. Plasma transudates enter into the airways in inflammatory condition because of increase in pulmonary vascular permeability. Fibrinogen in the exuded plasma is activated by tissue factor expressed on surface of activated alveolar macrophages and epithelial cells, which thereafter results in clot formation in the airways. Intrabroncheal fibrin plays several roles in the pulmonary pathology. First, it blocks ventilation. According to the current invention we have shown that more than 40% of bronchi and bronchioles were obstructed by fibrin-containing cast after smoke inhalation and pneumonia in sheep. When some part of lung is obstructed, gas exchange falls because of ventilation / per mismatch. If the patients were mechanically ventilated, the ventilated part of lung would be overstretched and it would be a cause of barotraumas. Secondly, fibrin inhibits surfactant activity also limiting lung function. It is known that heparin nebulization is effective to prevent airway obstruction and subsequent acute lung injury. However, there is a report suggesting that the administration of heparin alone, in a nebulize form, was not effective in smoke inhalation-induced acute lung injury (ALI). We think that the reason for this discrepancy is because heparin's anti-coagulant potential is antithrombin dependent and the antithrombin activity was not properly taken into account in other work, and that in this injury model antithrombin levels typically drop well below normal physiologic levels.

Another type of lung injury ARDS (acute respiratory distress syndrome) is a severe form of lung injury that occurs in approximately one tenth of U.S. critical care patients, it is a major contributor to ICU morbidity and mortality. The pathophysiology of ARDS includes inflammatory, vascular leak, uncontrolled coagulation and fibro-proliferative components. Local expression of thrombin and factor Xa enzymatic activity in the early ARDS lung causes intra-alveolar fibrin deposition and hyaline membrane formation that then contribute to impaired pulmonary function. Through its growth factor activity thrombin may also promote the development of lung fibrosis, which is a problem in later stages of ARDS. According to the current invention, models of acute lung injury have been used to investigate the role that thrombin plays in ARDS evolution and in its progression to multiple organ failure and eventual death in many patients.

Antithrombin is a broad-spectrum serine protease inhibitor. ATIII is a serpin with potent anti-coagulant activity. Rates of ATIII-mediated thrombin and factor Xa inhibition increase over 1000-fold upon binding to pharmaceutical heparin or vascular heparan sulfate proteoglycans. ATIII inhibits thrombin by forming a thrombin-ATIII complex that is removed from the circulation by the reticulo-endothelial system. ATIII has further inhibitory action on all known coagulation pathways. ATIII can also, together with heparin to inactivate TF-factor VII complexes. AT (antithrombin III) inhibits thrombin and fibrin formation and reduces the expression of adhesion molecules by endothelial cells. APC (activated protein C) inhibits F VIIIa and F Va, reduces endothelial cells and monocyte TF expression, inhibits PAI-1 and TAFI (thrombin-activatable fibrino-lysis inhibitor) by reducing thrombin generation. Recombinant human soluble thrombomodulin (rhs-TM) is able to convert protein C in its activated form (APC).

ATIII contains a heparin-binding domain at its active site. Heparin, other glycos-aminoglycans, and proteoglycans are expressed on endothelial surfaces and bind ATIII with high affinity. To inactivate thrombin, ATIII must bind to heparin. In the absence of heparin, AT binds to endothelial surfaces and functions as an anticoagulant, but it also stimulates prostacyclin release. Prostacyclin is a potent inhibitor of platelet aggregation, inhibits synthesis of pro-inflammatory cytokines,and attenuates neutrophil activation and subsequent release of elastase and oxygen free radicals.

Heparin accelerates antithrombins' inhibitory reaction several thousand fold, Thus, without proper levels of antithrombin in the airways, aerosolized heparin would not inhibit fibrin formation leading to little if any improvement in treatment of lung injury by smoke or bums. According to the current invention we demonstrate that antithrombin nebulization in conjunction with heparin delivery is an effective and reliable in treatment for ALI from a smoke and/or bum induced injury and claim methods of treating such lung injuries more effectively.

Several human and animal studies have examined the effects of modulating the coagulation cascade in acute and chronic lung disease. According to the current invention, the exogenous delivery of the highly specific direct thrombin inhibitor, antithrombin III, is protective in animal models of ALI. In addition, heparin, which inhibits coagulation proteases by potentiating the formation of antithrombin III-serine protease complexes, leads to improved gas exchange in an animal model of ALI. Heparin has also been shown to attenuate bleomycin-induced pulmonary fibrosis in mice. Therefore concurrent administration of the two molecules has a synergistic and positive effect on ALI patients.

In LPS-induced lung injury, intravenous ATIII (250 units/kg) has been shown to reduce vascular injury, leukocyte accumulation, and vascular permeability. Heparin, alone may not be able to fully or optimally prevent lung damage. ALI and ARDS are associated with increased pro-coagulant and reduced fibrinolytic activities in alveoli and interstitial spaces in the lung. Fibrin deposition, which is the hallmark of early phase ALI, is accompanied by increased levels of PAI-1 and neutrophil accumulation in the lung. Fibrin deposition and neutrophil elastase-induced digestion to fibrin degradation products stimulate fibroblast aggregation and collagen secretion, leading to pulmonary fibrosis. Whereas animal studies have demonstrated a consistent reduction in lung injury with the administration of anti-coagulants, such as TFPI, TF-factor VIIai, ATIII, soluble TM, and APC, clinical studies have been less convincing. ATIII administration has shown some benefits on lung injury due to smoke inhalation and bums.

Airway obstruction by fibrin cast is a serious problem in the smoke inhalation-induced acute lung injury (ALI). Prevention of airway casts may be important for clinical outcome. We report herein that ATIII and heparin jointly administered is an effective to prevent airway obstruction in an ovine smoke inhalation model. Moreover, the current invention teaches that the use of both heparin and antithrombin (ATIII), in a nebullized form and used for the treatment of lung injury act synergistically to improve gas exchange during experimental respiratory distress syndrome. ATIII and Heparin may also be useful for treating sepsis-induced coagulopathy. As previously stated heparin potentiates the naturally occurring plasma protease inhibitor, antithrombin, to inhibit thrombin, factor Xa, and other coagulation enzymes. When ATIII is supplied along with heparin this combination acts synergistically to inhibit thrombin and fibrin deposition.

### SUMMARY OF THE INVENTION

The invention is based, in part, on the discovery that aerosolized antithrombin III (ATIII) is effective in treating lung disorders, e.g., lung inflammation and injury. Through the instant invention it has been determined that the prevention of the fibrin clots by aerosolized anticoagulants improves pulmonary lung function with burn and smoke inhalation injuries.

Thus, administration of both heparin and ATIII by inhalation provides more efficient treatment of lung disorders, e.g., lung inflammation and injury, than intravenous administration.

According to a preferred embodiment of the current invention antithrombin and heparin, and specifically aerosolized versions of both antithrombin and heparin were found to be a more effective anticoagulant to prevent the airway obstruction than heparin or other agents alone. This was also found to be true when aerosolized antithrombin and heparin were tested against a synthetic thrombin inhibitor, in smoke inhalation induced ALI model in sheep.

Both antithrombin and heparin nebulization inhibited the airway obstruction and attenuated the gas exchange. In addition to that, antithrombin seems to have an anti-inflammatory effect, which results in the attenuation of lung inflammation and subsequent edema formation when challenged with smoke and/or burn injury.

Accordingly, in one aspect, the invention features a method of treating a subject having a lung disorder, e.g., lung inflammation and/or injury, which includes administration of a therapeutically effective amount of both ATIII and heparin by inhalation. The lung disorder can be an acute or chronic lung disorder. In one embodiment, the lung disorder is an acute lung injury, e.g., septic acute lung injury or ARDS, or airway blockage. Lung injury and/or inflammation can be in response to, e.g., exposure to an external agent, e.g., a viral agent (e.g., Pseudomonas pneumonia), smoke or asbestos. In other embodiments, the lung disorder can be, e.g., lung or pleural neoplasia, interstitial lung disease and/or organizing pleuitis.

In one embodiment, the composition of heparin and ATIII is administered using a jet aerosol or ultrasonic nebulizer system, or by a dry powder inhalation system. Such systems for aerosol administration of combination compositions are known.

In one embodiment, the ATIII is human ATIII. The ATIII can be naturally derived, e.g., from plasma, or recombinantly produced. Plasma derived ATIII is commercially available. In a preferred embodiment, the antithrombin III is transgenically produced, e.g., the ATIII is obtained from milk from a transgenic diary animal, e.g., a cow, a goat, a rabbit, or a mouse. Methods of producing ATIII in the milk of a transgenic animal are described in U.S. Patent Number 5,843,705, the contents of which is incorporated herein by reference. Likewise, heparin can be naturally derived, e.g., from plasma, or recombinantly produced. Plasma derived heparin is commercially available. In a preferred embodiment, the heparin is produced recombinantly.

In a preferred embodiment, the subject is administered an aerosol composition that includes ATIII, heparin and a pharmaceutically acceptable carrier. Examples of pharmaceutically acceptable carriers include water and saline.

In one embodiment, the subject is periodically administered both ATIII and heparin by inhalation, e.g., the subject is administered ATIII and heparin at regular intervals depending upon the pharmokinetics of each molecule. For example, the subject can be administered aerosol ATIII and heparin at the onset of lung inflammation and/or injury and then at set intervals after the initial administration, e.g., ATIII and/or heparin can be administered by inhalation every hour, 2 hours, 3 hours, 4 hours, 6 hours, twice a day, or three, four, five, six times a day dependent upon the patients need and the pharmacologically profile of the individual agents. The period of administration can be over a period of about 24, 48, 72, 96, 120, 144 or 168 hours. In another embodiment, the subject is administered the ATIII and heparin combination by inhalation as needed, e.g., ATIII is administered upon indication of one or more continued or reoccurring symptom(s) of lung inflammation or injury.

An effective dose of ATIII, e.g., transgenically produced ATIII, can be between about 1-150 U/kg, 25-125 U/kg, 50-100 U/kg, or 60-75 U/kg of body weight. In another aspect, an effective dose can be greater than about 1 mg/kg, 5 mg/kg, 10 mg/kg, but less than about 150 mg/kg, 100 mg/kg, 70 mg/kg.

An effective dose of the companion composition heparin, can be between about 1-30 U/kg, 2-25 U/kg, 50-100 U/kg, or 60-75 U/kg of body weight. In another aspect, an effective dose can be greater than about 1 mg/kg, 5 mg/kg, 10 mg/kg, but less than about 150 mg/kg, 100 mg/kg, 70 mg/kg.

In a preferred embodiment, the dose of aerosol for the ATIII & heparin combination is less than 10%, 20%, 30%, 40%, 50%, 60% the dose of ATIII & heparin intravenously administered to treat the same disorder, e.g., to have the same effect on one or more symptom of lung inflammation or injury.

According to the current invention both antithrombin and heparin nebulization inhibited the airway obstruction and attenuated the gas exchange. In addition to that, antithrombin had an additional and enhanced anti-inflammatory effect, which results in the improved attenuation of lung inflammation and subsequent edema formation. Moreover, there is a synergistic effect when antithrombin and heparin are used together to treat lung injury due to smoke inhalation and/or bums.

In another embodiment, the invention features a kit for treating lung injuries. Preferably, the kit includes a therapeutically effective amount of an aerosol form of ATIII, and instructions for use. Preferably, the aerosol further includes a pharmaceutically acceptable carrier. Examples of pharmaceutically acceptable carriers include water and saline.

In one embodiment, an effective dose of ATIII, e.g., transgenically produced ATIII, can be between about 10-300 U/kg, 25-125 U/kg, 50-100 U/kg, or 60-75 U/kg of body weight. In another aspect, an effective dose can be greater than about 1 mg/kg, 5 mg/kg, 10 mg/kg, but less than about 150 mg/kg, 100 mg/kg, 70 mg/kg.

In a preferred embodiment, the kit is a kit for treating an acute or chronic lung disorder. Preferably, the lung disorder is an acute lung injury, e.g., septic acute lung injury or acute respiratory distress syndrome (ARDS). Lung injury and/or inflammation can be in response to, e.g., exposure to an external agent, e.g., a viral agent (e.g., *Pseudomonas pneumonia*), smoke or asbestos. In other embodiments, the lung disorder can be, e.g., lung or pleural neoplasia, interstitial lung disease and/or organizing pleuitis.

In one embodiment, the kit includes the ATIII & heparin composition in a jet aerosol or ultrasonic nebulizer system, or a dry powder inhalation system.

In one embodiment, the kit includes an aerosol form of human ATIII and heparin. The ATIII can be naturally derived, e.g., from plasma, or recombinantly produced. In a preferred embodiment, the antithrombin III is transgenically produced, e.g., the ATIII is obtained from milk from a transgenic diary animal, e,g., a cow, a goat, a rabbit, or a mouse. This while the heparin of the invention is typically derived from a recombinant *in vitro* source.

The details of one or more embodiments of the invention are set forth in the accompanying drawings and the description below. Other features, objects, and advantages of the invention will be apparent from the description and drawings, and from the claims.

### DESCRIPTION OF DRAWINGS

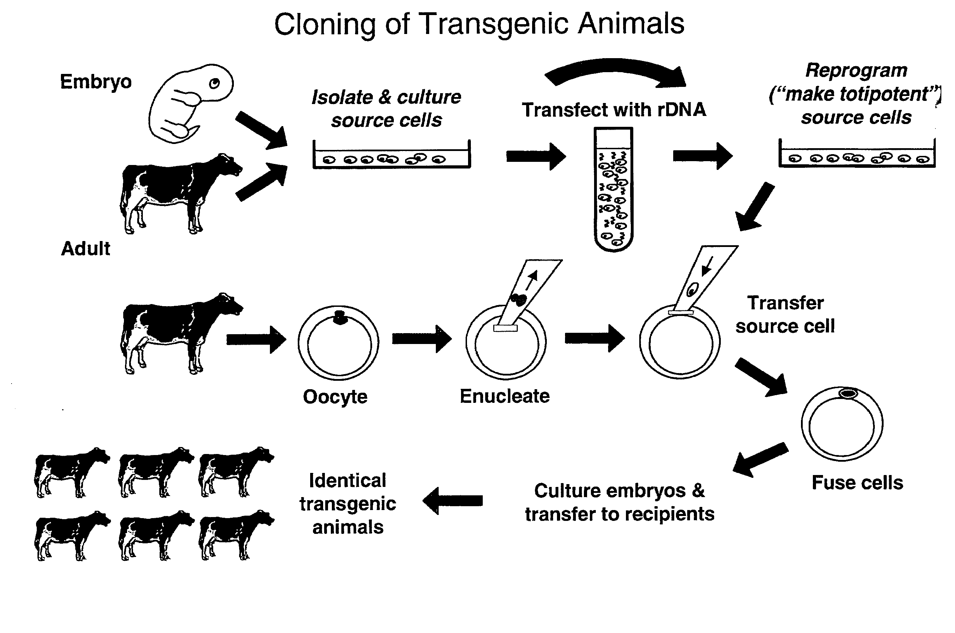

FIG. 1 Shows a Generalized Diagram of the Process of Creating Cloned Animals through Nuclear Transfer.

### DETAILED DESCRIPTION

The following abbreviations have designated meanings in the specification:

| **Abbreviation Key**: | | |
|---|---|---|
| | Somatic Cell Nuclear Transfer | (SCNT) |
| | Nuclear Transfer | (NT) |
| | Synthetic Oviductal Fluid | (SOF) |
| | Fetal Bovine Serum | (FBS) |
| | Polymerase Chain Reaction | (PCR) |
| | Bovine Serum Albumin | (BSA) |

### Explanation of Terms:

### Bovine - Of or relating to various species of cows.

Biological Fluid - an aqueous solution produced by an organism, such as a mammal, bird, amphibian, or reptile, which contains proteins that are secreted by cells that are bathed in the aqueous solution. Examples include: milk, urine, saliva, seminal fluid, vaginal fluid, synovial fluid, lymph fluid, amniotic fluid, blood, sweat, and tears; as well as an aqueous solution produced by a plant, including, for example, exudates and guttation fluid, xylem, phloem, resin, and nectar.
Biological-fluid producing cell - A cell that is bathed by a biological fluid and that secretes a protein into the biological fluid.
Biopharmaceutical - shall mean any medicinal drug, therapeutic, vaccine or any medically useful composition whose origin, synthesis, or manufacture involves the use of microorganisms, recombinant animals (including, without limitation, chimeric or transgenic animals), nuclear transfer, microinjection, or cell culture techniques.
Caprine - Of or relating to various species of goats.
Encoding - refers generally to the sequence information being present in a translatable form, usually operably linked to a promoter. A sequence is operably linked to a promoter when the functional promoter enhances transcription or expression of that sequence. An anti-sense strand is considered to also encode the sequence, since the same informational content is present in a readily accessible form, especially when linked to a sequence which promotes expression of the sense strand. The information is convertible using the standard, or a modified, genetic code.
Expression Vector - A genetically engineered plasmid or virus, derived from, for example, a bacteriophage, adenovirus, retrovirus, poxvirus, herpesvirus, or artificial chromosome, that is used to transfer an ATIII protein coding sequence, operably linked to a promoter, into a host cell, such that the encoded recombinant ATIII protein is expressed within the host cell.
Functional Proteins - Proteins which have a biological or other activity or use, similar to that seen when produced endogenously.
Slide - A glass slide for parallel electrodes that are placed a fixed distance apart. Cell couplets are placed between the electrodes to receive an electrical current for activation.
Homologous Sequences - refers to genetic sequences that, when compared, exhibit similarity. The standards for homology in nucleic acids are either measures for homology generally used in the art or hybridization conditions. Substantial homology in the nucleic acid context means either that the segments, or their complementary strands, when compared, are identical when optimally aligned, with appropriate nucleotide insertions or deletions, in at least about 60% of the residues, usually at least about 70%, more usually at least about 80%, preferably at least about 90%, and more preferably at least about 95 to 98% of the nucleotides. Alternatively, substantial homology exists when the segments will hybridize under selective hybridization conditions, to a strand, or its complement. Selectivity of hybridization exists when hybridization occurs which is more selective than total lack of specificity. Typically, selective hybridization will occur when there is at least about 55% homology over a stretch of at least about 14 nucleotides, preferably at least about 65%, more preferably at least about 75%, and most preferably at least about 90%.
Leader sequence or a "signal sequence" - a nucleic acid sequence that encodes a protein secretory signal, and, when operably linked to a downstream nucleic acid molecule encoding an ATIII protein and directs ATIII secretion. The leader sequence may be the native human ATIII leader, an artificially-derived leader, or may obtained from the same gene as the promoter used to direct transcription of the ATIII coding sequence, or from another protein that is normally secreted from a cell.
Milk-producing cell - A cell (e.g., a mammary epithelial cell) that secretes a protein into milk.
Milk-specific promoter - A promoter that naturally directs expression of a gene in a cell that secretes a protein into milk (e.g., a mammary epithelial cell) and includes, for example, the casein promoters, e.g., alpha casein promoter (e.g., alpha S-1 casein promoter and alpha S2-casein promoter), beta casein promoter (e.g., the goat beta casein gene promoter (DiTullio, BIOTECHNOLOGY 10:74-77, 1992), gamma casein promoter, and kappa casein promoter; the whey acidic protein (WAP) promoter (Gorton et al., BIOTECHNOLOGY 5: 1183-1187, 1987); the beta-lactoglobulin promoter (Clark et al., BIOTECHNOLOGY 7: 487-492, 1989); and the alpha-lactalbumin promoter (Soulier et al., FEBS LETTS. 297:13, 1992). Also included are promoters that are specifically activated in mammary tissue and are thus useful in accordance with this invention, for example, the long terminal repeat (LTR) promoter of the mouse mammary tumor virus (MMTV).
Nuclear Transfer - refers to a method of cloning wherein the nucleus from a donor cell is transplanted into an enucleated oocyte.
Operably Linked - A gene and one or more regulatory sequences are connected in such a way as to permit gene expression when the appropriate molecules (e.g., transcriptional activator proteins) are bound to the regulatory sequences.
Ovine - of, relating to or resembling sheep.
Parthenogenic - The development of an embryo from an oocyte without the penetration of sperm.
Pharmaceutically Pure - Refers to protein that is suitable for unequivocal biological testing as well as for appropriate administration to effect treatment of a human patient. Substantially pharmaceutically pure means at least about 90% pure.
Porcine - of or resembling pigs or swine.
Promoter - A minimal sequence sufficient to direct transcription, Also included in the invention are those promoter elements which are sufficient to render promoter-dependent gene expression controllable for cell type-specific, tissue-specific, temporal-specific, or inducible by external signals or agents; such elements may be located in the 5' or 3' or intron sequence regions of the native gene.
Protein - as used herein is intended to include glycoproteins, as well as proteins having other additions. This also includes fragmentary or truncated polypeptides that retain physiological function.
Therapeutically-effective amount - An amount of a therapeutic molecule or a fragment thereof that, when administered to a patient, inhibits or stimulates a biological activity modulated by that molecule.
Transformation, "Transfection," or "Transduction" - Any method for introducing foreign molecules into a cell. Lipofection, DEAE-dextran-mediated transfection, microinjection, nuclear transfer (see, e.g., Campbell et al. BIOL. REPROD. 49:933-942, 1993; Campbell et al., NATURE 385:810-813, 1996), protoplast fusion, calcium phosphate precipitation, transduction (e.g., bacteriophage, adenoviral retroviral, or other viral delivery), electroporation, and biolistic transformation are just a few of the methods known to those skilled in the art which may be used,
Transformed cell or Transfected cell - A cell (or a descendent of a cell) into which a nucleic acid molecule encoding ATIII has been introduced by means of recombinant DNA techniques. The nucleic acid molecule may be stably incorporated into the host chromosome, or may be maintained episomally.
Transgene - Any piece of a nucleic acid molecule that is inserted by artifice into a cell, or an ancestor thereof, and becomes part of the genome of the animal which develops from that cell. Such a transgene may include a gene which is partly or entirely exogenous (i.e., foreign) to the transgenic animal, or may represent a gene having identity to an endogenous gene of the animal.
Transgenic - Any cell that includes a nucleic acid molecule that has been inserted by artifice into a cell, or an ancestor thereof, and becomes part of the genome of the animal which develops from that cell.
Transgenic Organism - An organism into which genetic material from another organism has been experimentally transferred, so that the host acquires the genetic information of the transferred genes in its chromosomes in addition to that already in its genetic complement.
Ungulate - of or relating to a hoofed typically herbivorous quadruped mammal, including, without limitation, sheep, swine, goats, cattle and horses.
Vector - As used herein means a plasmid, a phage DNA, or other DNA sequence that (1) is able to replicate in a host cell, (2) is able to transform a host cell, and (3) contains a marker suitable for identifying transformed cells.

According to the present invention, it was found that the use of an aerosol form of ATIII & Heparin reduced ALI at lower doses than intravenously administered ATIII alone or heparin alone. Accordingly, the invention features aerosol formulations including ATIII, as well as, methods of using such aerosol forms of ATIII to treat a subject having a lung disorder, e.g., lung injury or inflammation.

Recombinant human ATIII and heparin nebulized and delivered together, were an effective in preventing airway obstruction as well as improving the gas exchange after smoke inhalation injury followed by pneumonia. Further more, ATIII reduced the lung inflammation and subsequent edema formation. The amount of ATIII was less than that effective in intravenous administration. According to the data presented herein and according to a preferred embodiment of the instant invention there was no adverse effect observed including bleeding tendency. Therefore the use of aerosolized ATIII & heparin nebulization is a new and more effective treatment strategy for the treatment of smoke inhalation than either compound alone.

The term "treat" or "treatment" as used herein refers to alleviating or reducing one or more symptom(s) associated with a lung disorder. For example, symptoms of lung injury and/or inflammation include: 1) reduced pulmonary gas exchange; 2) reduced pulmonary shunt fraction; 3) extracellular fibrin deposition; 4) increased vascular permeability; 5) decreased lipoprotein surfactant deposition; 6) tissue remodeling; 7) coagulation; and/or 8) increased alveolar tension. As used herein, an amount of an aerosolized form of ATIII effective to treat a lung disorder, or a "therapeutically effective amount" refers to an amount of ATIII aerosol which is effective, upon single or multiple dose administration to a subject, in curing, alleviating, relieving or improving a subject with a lung disorder as described herein beyond that expected in the absence of such treatment.

The ATIII & heparin combination can be administered as a dry powder formulation, or with a pharmaceutically acceptable carrier. Pharmaceutically acceptable carriers include, e.g., sterile water, saline and alcohols. The pharmaceutical ATIII & heparin aerosol composition can further include other therapeutic agents (e.g., other agents which alleviate or reduce lung inflammation or injury), or other pharmaceutical adjuvants, diluents, etc. The composition of the invention can be administered, e.g., as a complex with, or encapsulated in a liposome.

For administration by inhalation, the compounds of the invention can be delivered in the form of an aerosol spray from pressured container or dispenser which contains a suitable propellant, e.g., a gas such as carbon dioxide, or a nebulizer. As used herein, the term "aerosols" refers to dispersions in air of solid or liquid particles, of fine enough particle size and consequent low settling velocities to have relative airborne stability (See Knight, V., VIRAL AND MYCOPLASMAL INFECTIONS OF THE RESPIRATORY TRACT. 1973, Lea and Febiger, Phila. Pa., pp. 2).

The nebulization of ATIII or heparin may be achieved by a gas pressure or by ultrasound. Generally speaking, a nebulizer is an apparatus permitting the administration of aerosols. The nebulizers may be of any type and their structures are known to a person skilled in the art, and these devices are commercially available. The aerosols of the invention can be made by nebulizing an ATIII & heparin containing solution using a variety of known nebulizing techniques. One nebulizing system is the "wo-phase" system which consists of a solution or a suspension of active ingredient in a liquid propellant. Both liquid and vapor phases are present in a pressurized container and when a valve on the container is opened, liquid propellant containing the solution or suspension is released. This can result in fine aerosol mist or aerosol wet spray.

There are a variety of nebulizers that are available to produce aerosols including small volume nebulizers. Compressor driven nebulizers incorporate jet technology and use compressed air or medical oxygen to generate the aerosol. Commercially available devices are available from Healthdyne Technologies Inc; Invacare Inc.; Mountain Medical Equipment Inc.; Pari Respiratory Inc.; Mada Medical Inc.; Puritan-Bennet; Schuco Inc.; Omron Healthcare Inc.; DeVilbiss Health Care Inc; and Hospitak Inc. Ultrasonic nebulizers, e.g., an ultrasonic type nebulizer with a quartz crystal vibrating at high frequency, can also be used to deliver the ATIII // heparin composition.

Toxicity and therapeutic efficacy of such ATIII aerosols can be determined by standard pharmaceutical procedures in cell cultures or experimental animals, *e.g*., for determining the LD₅₀ (the dose lethal to 50% of the population) and the ED₅₀ (the dose therapeutically effective in 50% of the population). The dose ratio between toxic and therapeutic effects is the therapeutic index and it can be expressed as the ratio LD₅₀/ED₅₀. Compounds which exhibit high therapeutic indices are preferred. While compounds that exhibit toxic side effects may be used, care should be taken to design a delivery system that targets such compounds to the site of affected tissue in order to minimize potential damage to uninfected cells and, thereby, reduce side effects.

The data obtained from the cell culture assays and animal studies can be used in formulating a range of dosage for use in humans. The dosage of such compounds lies preferably within a range of circulating concentrations that include the ED₅₀ with little or no toxicity. The dosage may vary within this range depending upon the dosage form employed and the route of administration utilized. For any compound used in the method of the invention, the therapeutically effective dose can be estimated initially from cell culture assays. A dose may be formulated in animal models to achieve a circulating plasma concentration range that includes the IC₅₀ (i.e., the concentration of the test compound which achieves a half-maximal inhibition of symptoms) as determined in cell culture. Such information can be used to more accurately determine useful doses in humans. Levels in plasma may be measured, for example, by high performance liquid chromatography.

Other methods of determining the dosage of an ATIII & heparin joint composition will include measuring a subject's circulating ATIII levels prior to treatment with ATIII. Based on circulating ATIII levels, the dosage of ATIII can be adjusted to be 50%, 100%, 150%, 250%, 300% greater than initial circulating levels.

The amount of aerosol formulation administered will typically in the in range of about 10 U/kg to about 250 U/kg of body weight, preferably about 25 U/kg to about 175 U/kg of body weight.

The skilled artisan will appreciate that certain factors may influence the dosage and timing required to effectively treat a subject, including but not limited to the severity of the disease or disorder, previous treatments, the general health and/or age of the subject, and other diseases present. Moreover, treatment of a subject with a therapeutically effective amount of a protein, polypeptide, or antibody can include a single treatment or, preferably, can include a series of treatments.

### MATERIALS AND METHODS

### Materials

Human recombinant antithrombin (ATIII) was a gift from GTC Biotherapeutics (Framingham, MA). Recombinant heparin was purchased from Daiichi Pharmaceutical Co. (Tokyo, Japan). All other reagents were of analytical grade.

### Animal preparation

The Animal Care and Use Committee of The University of Texas Medical Branch approved the experiments reported in this manuscript. All animals were handled within the guidelines established by the American Physiological Society and the National Institutes of Health. Twenty four female sheep were surgically prepared for study under halothane anesthesia as previously described. After measurement of baseline cardiovascular data, sheep received a combination injury of smoke inhalation and bacteria instillation as previously described. This animal model shows severe acute lung injury as well as hyperdynamic cardiovascular responses, which mimics human septic cases.

Briefly, *P. aeruginosa* (5 x 10⁹/kg) was instilled intrabronchially after the cotton smoke exposure (4 sets of 12 breaths, < 40 °C). After the smoke inhalation and bacterial instillation, animals were randomly assigned for the two treatment groups, ATIII & Heparin nebulization group; ATIII nebulization group, heparin-nebulization group, and saline nebulization control group. All animals were mechanically ventilated with 100% oxygen throughout the 24-h study (Tidal volume 12-15ml/kg, 30 breaths/min, PEEP 7.5cmH₂O). Tidal volume was adjusted to maintain PaCO₂ normal. The Ringer's lactate solution was injected in sufficient volume to prevent the hematocrit from rising. Initially, 2 ml/kg/h of Ringer's lactate was infused, and we adjusted the in rate up to 10 ml/kg/h based on the hematocrit levels. The body weight (kg) of animals did not significantly vary from one animal to another.

### Treatment

Recombinant human antithrombin (rhAT) and recombinant heparin were dissolved in distilled water. Then, the ATIII/heparin solution was mixed with saline and mixed solution containing ATIII & heparin and the animal was nebulized every 4 hrs. ATIII and Heparin was were separately mixed with saline and were also nebulized every 4 hrs. The control group received 15 ml saline nebulization as the same fashion. The solutions were nebulized by an ultrasonic nebulizer (Ultra-Neb99, DeVilbiss Co., Somerset, PA), which was connected to the tracheobronchial tree as shown previously. The size of the particles obtained by the nebulizer is reported less than 4 microns. The nebulization was started one hour after the insult and every 4 hrs thereafter.

### Measurement of plasma NO₂ l NO₃ (NOx).

The concentration of NOx (total amount of nitric oxide metabolites) in plasma was measured with a chemiluminescent NO analyser (Antek, model 7020) as previously described.

### Blood culture and Coagulation parameters

Arterial blood was cultured to test the bacteremia (Septi-check, Becton Dickinson, Sparks, MD). Heparinized blood samples were carefully withdrawn from the femoral artery catheter at baseline and at 3, 6, 12, and 24 hrs.

Activated clotting time was monitored at baseline, 12h and 24h after the injury using Hemochron model 801 (International Technidyne Co., Edson, NJ).

Citrated plasma was collected at baseline, 6h, and 24h after the injury for the antithrombin assay. The activities of antithrombin was measured using a chromogenic substrates (S-2765, Diagnostica Stago, Parsippany, NJ). Data were expressed as % of normal standard plasma.

### Airway pressures

Peak and plateau airway pressures were monitored by a ventilator (Servo Ventilator 900C, Seimens-Elema, Sweden). Since peak airway pressure reflects the sum of airway resistance and compliance, and the plateau pressure reflects mainly the airway compliance, we subtracted the plateau pressure from the peak pressure (Δ airway pressures). Thus, Δ airway pressure is an index of airway resistance.

### Statistical analysis

The statistical software StatView 5.0 (SAS Institute, Cary, NC) was used to perform the analysis. Data are expressed as means ± SEM. Repeated measured analysis of variance (ANOVA) or ANOVA with Tuckey's post hoc test was used to compare data among the groups. Where appropriate, inter-group comparisons at individual time points were made using the Student Newman-Keuls test for unpaired data. In the histological study, a non-parametric Mann-Whitney U test was performed. A p-value < 0.05 was considered statistically significant.

### Methods for Joint Composition

Female sheep were surgically prepared for chronic study. After recovery, a tracheostomy was performed under ketamine/halothane anesthesia, and given a bum (40% of total body surface, 3^{rd} degree) and inhalation injury (cotton smoke). Sheep were given 4 ml/kg/24h of Ringer's lactate and mandatory ventilation. Sheep were divided into 5 groups: 1) non-injured, non-treated (sham, n=6); injured, nebulized with 2) saline (saline, n=6), 3) heparin (Hep, n=5), 4) antithrombin (AT, n=5), and hep+AT (n=5). Saline, heparin (10,000u), and antithrombin (290u) were nebulized every 4h beginning 2h after the injury. Experiment lasted 48h.

### Results and Conclusion

The cardiopulmonary variables were stable in sham animals. The saline group showed marked signs of acute lung injury evidenced by deteriorated gas exchange [PaO/FiO, pulmonary shunt (Qs/Qt)], increased lung lymph flow associated with increased airway pressures. Post treatment with heparin or antithrombin alone had no noticeable effects on these changes. However, combined heparin and antithrombin therapy reversed those alterations. The antithrombin concentration in bronchi-alveolar lavage in saline nebulized sheep was decreased. Taken together, the results indicated that administration of the joint composition prevented fibrin clot retention and relieved airway obstruction thereby improving airway clearance, improving pulmonary function in sheep with combined bum and smoke inhalation injury. Antithrombin and heparin exert stronger effect when they are aerosolized as a combination, See Table I below.

**Table I.**

| | Sham | Saline | Heparin | AT | Heparin+AT |
|---|---|---|---|---|---|
| PaO2/FiO2 | | | | | |
| 0h | 510±9 | 517±14 | 533±8 | 512±12 | 520±7 |
| 48h | 560±60 | 149±31† | 151±22† | 145±27† | 304±53*† |
| Qs/Qt | | | | | |
| 0h | 0.18±0.01 | 0.13±0.03 | 0.15±0.01 | 0.15t0.02 | 0.19±0.02 |
| 48h | 0.12±0.01 | 0.38±0,06† | 0.34±0,01† | 0.44±0.05† | 0.22±0.03* |

| | | | | | |
|---|---|---|---|---|---|
| *p<0.05 vs. saline; †<0.05 vs. sham; | | | | | |

### RESULTS

The peak levels of carboxy-hemoglobin (%) in the saline-treated group, ATIII-treated group, and heparin-treated group suggested that all the groups received almost the same amount of smoke inhalation. No animals died during the 24-h study period.

### Blood Culture

The arterial samples were carefully withdrawn for the bacterial culture at the several time points during the study as indicated in the Method section. *P*. *aeruginosa* was negative in all the baseline samples. During the 24h study, *P*. *aeruginosa* was detected in 33% (3/10) in saline-control group but 0% in both ATIII and heparin groups. Although the percentage was lower in ATIII and heparin-treated groups, there were no statistical differences from the saline-treated group.

### Pulmonary gas exchange

The PaO₂/FiO₂ (P/F) ratio dropped significantly after the insult in all the groups. However, the drop was significantly less in rhAT-nebulization group and heparin-nebulization group (Fig. 1A). The attenuation in gas exchange by ATIII and heparin was about the same extent (Fig. 1A). The pulmonary shunt fraction significantly increased after the insult, but the increase was significantly less in ATIII or heparin nebulization groups (Fig. 1B).

### Airway pressures

Peak and plateau airway pressures were monitored by the ventilator during the study. Delta airway pressure (=[Peak pressure]-[Plateau pressure]) was calculated to analyze the airway resistance. Delta airway pressure gradually increased in saline-treated control. ATIII-treated group and heparin-treated group showed no increase in delta airway pressures, although there were not statistical differences between the groups.

### Lung histopathology

Twenty-four hours after the smoke and bacterial challenge, there was a marked inflammatory reaction in the affected sheep's lungs that was characterized by cellular infiltrates in the interstitium and the air spaces. The infiltrates were predominantly composed of neutrophils. Interstitial edema, vascular congestion and hemorrhage were also observed. The histology scores, based on the severity and extent of congestion, edema, inflammation, and hemorrhage, were significantly higher after exposure to smoke and bacteria. Our historical sham-injured sham-treated animal (n=7) showed 1.0 or less in all the individual histopathology scores.

Heparin slone showed almost the same scores as saline control. Interestingly, hemorrhage score was not higher but less in both ATIII-treated and heparin-treated group. The total histology score was calculated. Since each congestion, edema, inflammation, and hemorrhage was scored from 0 (normal) to 4 (intense), the maximum of total histology score was 16. It was significantly lower in ATIII-treated group. In contrast, heparin nebulization did not attenuate the total histology score. It was 3.3±0.7 in historical sham-injury group (n=7).

Bronchi and bronchioles were obstructed by infiltrates of neutrophils, shed bronchial epithelial cells, mucus and fibrin. Airway obstruction of more than 30% of the cross-sectional area was found at both the bronchial and bronchiolar levels in saline treated group. ATIII nebulization inhibited the airway obstruction significantly in bronchi levels. Heparin inhibited the obstruction significantly in both bronchi and bronchiole levels.

### Lung wet/dry weight ratio

Lung wet/dry weight ratios increased significantly 24 h after smoke inhalation followed by bronchial instillation of bacteria compared to the historical sham injury's data (wet/dry ratio=3.70±0.17, n=7). This increase was significantly attenuated by ATIII nebulization but not by heparin alone.

### Changes in nitrate/nitrite (NOx) levels

Plasma NOx levels increased 2-3 folds of baseline levels after the insult. ATIII group showed the similar increase in initial 3h but significantly less in later phase until 24 h (Fig.6). Heparin seemed to be lower in NOx levels during 6-15 hrs but it reached to the same extent as the saline-treated group later on. There was not a statistical difference between heparin-treated group vs. saline-treated group.

### Blood count and coagulation parameters

The baseline leukocyte and platelet numbers were not different between the groups. The numbers of leukocytes fell significantly 24 h after the injury.

### DISCUSSION

Fibrin formation in the airway is known as a hallmark of acute/chronic lung injury. In septic condition, vascular permeability increases and fibrinogen-containing plasma comes into the airways, where tissue factors are expressed by the stimulation of cytokines. Therefore, fibrinogen easily makes a clot in the airways. Fibrin formation in the airway is a cause of lung injury by two means. First, it blocks the part of airways and inhibits the gas exchange. Second, fibrin inhibits the surfactant activity and it would be a cause of atelectasis. When the patients or animals are mechanically ventilated, the airway obstruction also causes ventilator induced lung injury. Also, overstretching the ventilated part of lung induces chemokines such as interleukin 8.

We have shown that heparin nebulization is effective in preventing airway obstruction and improving gas exchange. We determined that heparin nebulization prevents fibrin clot formation and attenuates acute lung injury. However, heparin's anticoagulant property is antithrombin dependent. According to the current invention, smoke inhalation, lung injury and pneumonia were initiated in laboratory animals designed to mimic what occurs in human occurrences of the same injuries or disease states. According to the current model, plasma antithrombin activity was measured and compared.

According to a preferred embodiment of the current invention, the baseline values of plasma antithrombin activity decreased gradually after the injury, and reached to approximately 50 % at 24 hrs. Similar phenomenon is reported in bum or sepsis patients. In such conditions, heparin's effect is not seen. According to the instant claims we provide that the infusion of ATIII was effective in treating lung injury. Based on this intravenous study, it was determined that a lower dosage range of ATIII directly by a nebulizer into the airways would be effective as well. This is the reason we designed the present study. Also, we calculated the amount (moles) of aerosolized ATIII that we administered and tested the effect of heparin of approximately the same anti-thrombotic potential.

Antithrombin is one of the major physiologic anticoagulants. Besides inhibiting various coagulation factors such as thrombin, FXa, FXIa, FXIIa, and FIXa, antithrombin is known to have some anti-inflammatory aspects. Various animal and clinical studies showed that intravenous antithrombin was beneficial in sepsis, endotoxin shock, renal ischemia-reper injury, and bums. Two mechanisms of action are now considered. First, antithrombin promotes prostacyclin release from endothelial cells. Since prostacyclin inhibits cytokine production, adhesion molecule expression, and platelet aggregation, antithrombin inhibits the inflammatory reactions indirectly via prostacyclin. Second, antithrombin receptor called syndecan-4 was found recently. Once antithrombin binds to syndecan-4, it inhibits nuclear factor kappa B translocation and inhibits the inflammatory signal transduction in leukocytes and endothelial cells. Thus, antithrombin directly alters inflammatory processes via inhibition of NF-kappa B activation.

Heparin was designed to inhibit thrombin activity and the specificity is very high. Compare to antithrombin, heparin is a very small molecule (molecular weight is 527 Da) and directly inhibits thrombin. There are few reports showing the anti-inflammatory effect of heparin. We thought these differences between antithrombin and heparin might show different effects on inhalation injury. In the present study, both ATIII and heparin inhibited the airway obstruction following smoke inhalation and pneumonia almost to the same extent, see Table I. As a consequence, pulmonary gas exchange was attenuated by both ATIII and heparin. When used together they displayed a synergistic effect/ Regarding the airway obstruction, heparin was more effective in preventing cast formation than rhAT. Since heparin is smaller than ATIII and it is a non-protein material, it could be delivered better. However, according to the improvement afforded by the current invention the histopathological analysis showed that ATIII improved the histological changes but heparin did not. Consistent with this result, lung wet/dry weight ratio was improved by ATIII nebulization but not by heparin. From these observations, we can speculate that anticoagulant potential of both ATIII and heparin inhibited the airway fibrin formation and attenuated the gas exchange. In addition to that, ATIII has an anti-inflammatory property and that improved the lung inflammation and subsequent lung edema formation. The drop in white cell count was less severe in ATIII-treated group compare to heparin-treated group, suggesting also that ATIII inhibited the inflammatory response.

As a part of the current invention both ATIII and heparin also reduced the incidence of septisemia. When the airways are obstructed, bacterial clearance would be interrupted and the closed non-ventilated area would be the bacterial bed. Therefore, we think the inhibition of airway obstruction is beneficial not only for the gas exchange but also for the prevention of the systemic bacterial translocation and sepsis.

The increase in plasma NOx, an index of nitric oxide (NO) formation, was significantly suppressed by aerosolized rhAT. NO is synthesized from 3 kinds of isoforms of nitric oxide synthase (NOS). Endothelial NOS (eNOS) and neuronal NOS (nNOS) are known as constitutive NOS (cNOS) and are expressed all the time. The third isoform is called inducible NOS (iNOS). I NOS is induced by inflammatory cytokines such as tissue necrosis factor-alpha. In the present study, ATIII inhibited the NO formation especially the latter 12 h of the experiment but did not inhibit it in the initial 12 hrs, suggesting that ATIII inhibits the iNOS expression. We have shown that iNOS is expressed not only from the alveolar macrophages but also alveolar epithelial cells. Since antithrombin is reported to inhibit NFkB activation, we have determined that nebulized ATIII inhibits iNOS expression from airway epithelial cells.

**Table II**

| | Changes in blood counts | |
|---|---|---|
| Time | Baseline | 24hrs |
| WBC (x10³/µl) | | |
| Saline | 6.19±0.71 | 1,85±0.46* |
| rhAT | 4.71±0.54 | 2.96±1.12 |
| Heparin | 6.15±0.93 | 2.08±0,81* |
| Platelets (x10³/µL) | | |
| Saline | 447±93 | 276±65* |
| rhAT | 456±52 | 246±41* |
| Heparin | 417±116 | 297±78 |

| | | |
|---|---|---|
| Mean ± SE. * p<0.05 vs. baseline | | |

**Table II Changes in activated clotting time**

| Time | Baseline | 24hrs |
|---|---|---|
| Saline | 151±8 | 168±7 |
| rhAT | 149±5 | 164±14 |
| Heparin | 147±2 | 157±5 |

| | | |
|---|---|---|
| Mean ± SE (sec) * p<0.05 versus saline | | |

Even though the total dose of ATIII was half of previously determined in intravenous studies (see Murakami (2001) AM. J. RESP. CRIT. CARE MED. 163:A553), the outcomes were more effective than intravenous administration. No adverse effects were observed. Thus, aerosolized ATIII was beneficial in septic acute lung injury following smoke inhalation and pneumonia in sheep.

A number of embodiments of the invention have been described. Nevertheless, it will be understood that various modifications may be made without departing from the spirit and scope of the invention. Accordingly, other embodiments are within the scope of the following claims.

In accordance with the methods of the current invention for transgenic animals a transgenic primary cell line (from either caprine, bovine, ovine, porcine or any other non-human vertebrate origin) suitable for somatic cell nuclear transfer is created by transfection of the protein nucleic acid construct of interest (for example, a mammary gland-specific transgene(s) targeting expression of a human alpha-fetoprotein - β-interferon protein to the mammary gland). The transgene construct can either contain a selection marker (such as neomycin, kanamycin, tetracycline, puromycin, zeocin, hygromycin or any other selectable marker) or be co-transfected with a cassette able to express the selection marker in cell culture.

The invention provides expression vectors containing a nucleic acid sequence described herein, operably linked to at least one regulatory sequence. Many such vectors are commercially available, and other suitable vectors can be readily prepared by the skilled artisan. "Operably linked" or "operatively linked" is intended to mean that the nucleic acid molecule is linked to a regulatory sequence in a manner which allows expression of the nucleic acid sequence by a host organism. Regulatory sequences are art recognized and are selected to produce the encoded polypeptide or protein. Accordingly, the term "regulatory sequence" includes promoters, enhancers, and other expression control elements which are described in Goeddel, GENE EXPRESSION TECHNOLOGY: METHODS IN ENZYMOLOGY 185, (Academic Press, San Diego, Calif. (1990)). For example, the native regulatory sequences or regulatory sequences native to the transformed host cell can be employed.

It should be understood that the design of the expression vector may depend on such factors as the choice of the host cell to be transformed and/or the type of protein desired to be expressed. For instance, the polypeptides of the present invention can be produced by ligating the cloned gene, or a portion thereof, into a vector suitable for expression in either prokaryotic cells, eukaryotic cells or both. **(**A LABORATORY MANUAL, 2nd Ed., ed. Sambrook et al. (Cold Spring Harbor Laboratory Press, 1989) Chapters 16 and 17)).

Following selection of colonies recombinant for the desired nucleic acid construct, cells are isolated and expanded, with aliquots frozen for long-term preservation according to procedures known in the field. The selected transgenic cell-lines can be characterized using standard molecular biology methods (PCR, Southern blotting, FISH). Cell lines carrying nucleic acid constructs of the bi-functional protein of interest, of the appropriate copy number, generally with a single integration site (although the same technique could be used with multiple integration sites) can then be used as karyoplast donors in a somatic cell nuclear transfer protocol known in the art. Following nuclear transfer, and embryo transfer to a recipient animal, and gestation, live transgenic offspring are obtained. Typically this transgenic offspring carries only one transgene integration on a specific chromosome, the other homologous chromosome not carrying an integration in the same site. Hence the transgenic offspring is heterozygous for the transgene, maintaining the current need for at least two successive breeding cycles to generate a homozygous transgenic animal.

### Purification of A TIII- Protein from a Biological Fluid

The ATIII protein of the invention may be purified from the biological fluid of a transgenic organism using standard protein purification techniques, such as affinity chromatography (see, e.g., Ausubel et al., CURRENT PROTOCOLS IN MOLECULAR BIOLOGY, John Wiley & Sons, New York, NY, 1998; *see also* Lubon et al., UNITED STATES PATENT: 5,831,141) or other methods known to those skilled in the art of protein purification. Once isolated, the ATIII- protein can, if desired, be further purified by e.g., by high performance liquid chromatography (HPLC; *e.g., see* Fisher, LABORATORY TECHNIQUES IN BIOCHEMISTRY AND MOLECULAR BIOLOGY, eds. Work and Burdon, Elsevier, 1980), and/or tangential flow filtration. Following purification, the ATIII protein is at least 80% pure, preferably 90% pure, more preferably 95% pure, and most preferably 99% pure.

### Animal Promoters

Useful promoters for the expression of ATIII in mammary tissue include promoters that naturally drive the expression of mammary-specific polypeptides, such as milk proteins, although any promoter that permits secretion of ATIII into milk can be used. These include, e.g., promoters that naturally direct expression of whey acidic protein (WAP), alpha S1-casein, alpha S2-casein, beta-casein, kappa-casein, beta-lactoglobulin, alpha-lactalbumin (see, e.g., Drohan et al., U.S. Patent No. 5,589,604; Meade et al., U.S. Patent No. 4, 873,316; and Karatzas et al., U.S. Patent No. 5,780,009), and others described in U.S. Patent No. 5,750,172. Whey acidic protein (WAP; Genbank Accession No. X01153), the major whey protein in rodents, is expressed at high levels exclusively in the mammary gland during late pregnancy and lactation (Hobbs et al., J. BIOL, CHEM. 257:3598-3605, 1982). For additional information on desired mammary gland-specific promoters, see, e.g., Richards et al., J. BIOL. CHEM. 256:526-532, 1981 (α-lactalbumin rat); Campbell et al., NUCLEIC ACIDS RES. 12:8685-8697, 1984 (rat WAP); Jones et al., J. BIOL. CHEM. 260:7042-7050, 1985 (rat β-casein); Yu-Lee & Rosen, J. BIOL. CHEM. 258:10794-10804, 1983 (rat γ-casein); Hall, BIOCHEM. J. 242:735-742, 1987 (human α-lactalbumin); Stewart, NUCLEIC ACIDS RES. 12:3895-3907, 1984 (bovine α-sl and κ-casein cDNAs); Gorodetsky et al., GENE 66:87-96, 1988 (bovine β-casein); Alexander et al., BUR. J. BIOCHEM. 178:395-401, 1988 (bovine κ-casein); Brignon et al., FEBS LETT. 188:48-55, 1977 (bovine α-S2 casein); Jamieson et al., GENE 61:85-90, 1987, Ivanov et al., BIOL. CHEM. Hoppe-Seyler 369:425-429, 1988, and Alexander et al., NUCLEIC ACIDS RES. 17:6739, 1989 (bovine β-lactoglobulin); and Vilotte et al., BIOCHIMIE 69:609-620, 1987 (bovine α-Iactalbumin).

The structure and function of the various milk protein genes are reviewed by Mercier & Vilotte, J. DAIRY SCI. 76:3079-3098, 1993. If additional flanking sequences are useful in optimizing expression, such sequences can be cloned using the existing sequences as probes. Mammary-gland specific regulatory sequences from different organisms can be obtained by screening libraries from such organisms using known cognate nucleotide sequences, or antibodies to cognate proteins as probes.

Useful signal sequences for expression and secretion of ATIII into milk are milk-specific signal sequences. Desirably, the signal sequence is selected from milk-specific signal sequences, i.e., from a gene which encodes a product secreted into milk. Most desirably, the milk-specific signal sequence is related to a milk-specific promoter described above. The size of the signal sequence is not critical for this invention. All that is required is that the sequence be of a sufficient size to effect secretion of ATIII, e.g., in the mammary tissue. For example, signal sequences from genes coding for caseins, e.g., alpha, beta, gamma, or kappa caseins, beta lactoglobulin, whey acidic protein, and lactalbumin are useful in the present invention. Signal sequences from other secreted proteins, e.g., proteins secreted by liver cells, kidney cell, or pancreatic cells can also be used.

Useful promoters for the expression of a recombinant polypeptide transgene in urinary tissue are the uroplakin and uromodulin promoters (Kerr et al., NAT. BIOTECHNOL. 16:75-79, 1998; Zbikowska, et al., BIOCHEM. J. 365:7-11, 2002; and Zbikowski et al., TRANSGENIC RES. 11:425-435, 2002), although any promoter that permits secretion of the transgene product into urine may be used.

A useful promoter for the expression and secretion of ATIII into blood by blood-producing or serum-producing cells (e.g., liver epithelial cells) is the albumin promoter (see, e.g., Shen et al., DNA 8:101-108, 1989; Tan et al., DEV. BIOL. 146:24-37, 991; McGrane et al., TIBS 17:40-44, 1992; Jones et al., J. BIOL. CHEM. 265:14684-14690, 1990; and Shimada et al., FEBS LETTERS 279:198-200, 1991), although any promoter that permits secretion of the transgene product into blood may be used.

Useful promoters for the expression of ATIII in semen are described in U,S. Patent No. 6,201,167. Useful avian-specific promoters are the ovalbumin promoter and the apo-B promoter. Other avian-specific promoters are known in the art. The ovalbumin promoter can be used to direct expression of ATIII that is then deposited in the egg white of the egg. The apo-B promoter can also be used to direct expression of a recombinant polypeptide in the liver, where it will eventually be deposited into the egg yolk. Avian eggs are an optimal vehicle for expressing large quantities of recombinant polypeptides for the following reasons: (1) a large amount of protein is packed into each egg, (2) eggs are easy to collect non-invasively and can be stored for extended periods of time, and (3) eggs are sterile and, unlike milk, do not contain bacterial contaminants. Specifically, for each egg, a bird can produce three grams of albumin in the oviduct, of which greater than 50% is ovalbumin. Another three grams is produced in the liver (serum lipoproteins) and deposited in the egg yolk. In addition, since birds do not typically recognize mammalian proteins immunologically because of their evolutionary distance from mammals, the expression of ATIII in birds is less likely to have any deleterious effect on the viability and health of the bird.

Other promoters that are useful in the methods of the invention include inducible promoters. Generally, recombinant proteins are expressed in a constitutive manner in most eukaryotic expression systems. The addition of inducible promoters or enhancer elements provides temporal or spatial control over expression of ATIII, and provides an alternative mechanism of expression. Inducible promoters include heat shock protein, metallothionien, and MMTV-LTR, while inducible enhancer elements include those for ecdysone, muristerone A, and tetracycline/ doxycycline.

The Tet-On and Tet-Off Gene Expression Systems (Clontech) is one example of an inducible system that is useful in the methods of the invention. This system uses a tetracycline (Tc) responsive element to maintain ATIII expression in either an on (constitutively off, induced with Tc) or off (constitutively on, repressed with Tc or doxycycline) mode. Selectable markers can also be incorporated into the ATIII transgene for easy identification of cells that have been transformed. Selectable markers generally fall into two functional categories: recessive and dominant. The recessive markers are usually genes that encode products that are not produced in the host cells (cells that lack the "marker" product or function). Marker genes for thymidine kinase (TK), dihydrofolate reductase (DHFR), adenine phosphoribosyl transferase (APRT), and hypoxanthine-guanine phosphoribosyl transferase (HGPRT) are in this category. Dominant markers include genes that encode products that confer resistance to growth-suppressing compounds (antibiotics, drugs) and/or permit growth of the host cells in metabolically restrictive environments. Commonly used markers within this category include a mutant DHFR gene that confers resistance to methotrexate; the gpt gene for xanthine-guanine phosphoribosyl transferase, which permits host cell growth in mycophenolic acid/xanthine containing media; and the neo gene for aminoglycoside 3'-phosphotransferase, which can confer resistance to G418, gentamycin, kanamycin, and neomycin.

### Nucleic Acid Vectors

In certain embodiments the invention concerns vectors, or recombinant expression vectors, comprising any of the nucleic acid molecules described herein. Vectors are used herein either to amplify DNA or RNA encoding proteins and/or to express DNA which encodes SSTR- proteins. Vectors include, but are not limited to, plasmids, phages, cosmids, episomes, viral particles or viruses, and integratable DNA fragments (i.e., fragments integratable into the host genome by homologous recombination). Viral particles include, but are not limited to, adenoviruses, baculoviruses, parvoviruses, herpesviruses, poxviruses, adeno-associated viruses, Semliki Forest viruses, vaccinia viruses, retroviruses, microparticles and naked DNA. In various embodiments, expression may be targeted to a particular cell type or cell population by a targeting ligand. Expression vectors include, but are not limited to, pcDNA3 (Invitrogen) and pSVL (Pharmacia Biotech). Other expression vectors include, but are not limited to, pSPORT^{™} vectors, pGEM^{™} vectors (Promega), pPROEXvectors^{™} (LTI, Bethesda, Md.), Bluescript^{™} vectors (Stratagene), pQE. ^{™} vectors (Qiagen), pSE420^{™} (Invitrogen), and pYES2^{™} (Invitrogen). Expression constructs may comprise a protein encoding polynucleotides operatively linked to an endogenous or exogenous expression control DNA sequence and a transcription terminator. Because of limited space for nucleic acid insertion in many vectors it may be desirable to insert smaller reporters or reporter constructs. For example, deletion of all or part of the somatosatin receptor carboxy terminus may be used. Expression control DNA sequences include promoters, enhancers, operators, and regulatory element binding sites generally, and are typically selected based on the expression systems in which the expression construct is to be utilized.

Promoter and enhancer sequences are generally selected for the ability to increase gene expression, while operator sequences are generally selected for the ability to regulate gene expression. Expression constructs of the invention may also include sequences encoding one or more selectable markers that permit identification of host cells bearing the construct. Expression constructs may also include sequences that facilitate homologous recombination in a host cell. In various embodiments constructs may also include sequences necessary for replication in a host cell.

Various exemplary tissue-specific promoters are listed herein (Pearse and Takor, 1979; Nylen and Becker, 1995). Although not a complete list, these promoters are exemplary of the types of promoters and enhancers that may be used in certain embodiments of the invention. Additional promoters, useful in the present invention, will be readily known to those of skill in the art.

Inducible promoters include but are not limited to MT II, MMTV (mouse mammary tumor virus), c-jun, Collagenase, Stromelysin, Murine MX Gene, GRP78 Gene, α-2-Macroglobulin, Vimentin, MHC Class I Gene H-2 kB, HSP70, Proliferin, Tumor Necrosis Factor and Thyroid Stimulating Hormone-α. Cell or tissue specific expression can be achieved by using cell-specific enhancers and/or promoters.
(*See generally,* Huber et al., ADV. DRUG DELIVERY REVIEWS 17:279-292, 1995).

Expression constructs may be utilized for production of an encoded protein, but may also be utilized simply to amplify an SSTR- protein encoding polynucleotide sequence. In some embodiments, the vector is an expression vector wherein the polynucleotide is operatively linked to a polynucleotide comprising an expression control sequence. In certain embodiments autonomously replicating recombinant expression constructs such as plasmid and viral DNA vectors incorporating polynucleotides. Expression vectors may be replicable DNA constructs in which a DNA sequence encoding SSTR- protein is operably linked or connected to suitable control sequences capable of effecting the expression of an SSTR- protein in a suitable host. DNA regions are operably linked or connected when they are functionally related to each other. For example, a promoter is operably linked or connected to a coding sequence if it controls the transcription of the sequence. Amplification vectors do not require expression control domains, but rather need only the ability to replicate in a host, usually conferred by an origin of replication, and a selection gene to facilitate recognition of transformants. The need for control sequences in the expression vector will vary depending upon the host selected and the transformation method chosen. Generally, control sequences include a transcriptional promoter, an optional operator sequence to control transcription, a sequence encoding suitable mRNA ribosomal binding and sequences that controls the termination of transcription and translation.

In various embodiments vectors may contain a promoter that is recognized by the host organism. The promoter sequences may be prokaryotic, eukaryotic, synthetic or viral. Examples of suitable prokaryotic sequences include the promoters of bacteriophage lambda (THE BACTERIOPAAGE LAMBDA, Hershey, A. D., Ed., Cold Spring Harbor Press, Cold Spring Harbor, N.Y. (1973); LAMBDA II, Hendrix, R. W., Ed., Cold Spring Harbor Press, Cold Spring Harbor, N.Y. (1980); and, Benoist et al., The trp, recA, heat shock, and lacZ promoters of E. coli and the SV40 early promote, NATURE, 290:304-310, (1981). Additional promoters include, but are not limited to, mouse mammary tumor virus, long terminal repeat of human immunodeficiency virus, maloney virus, cytomegalovirus immediate early promoter, Epstein Barr virus, Rous sarcoma virus, human actin, human myosin, human hemoglobin, human muscle creatine, and human metalothionein.

Additional regulatory sequences may also be included in vectors. Examples of suitable regulatory sequences are represented by the Shine-Dalgarno of the replicase gene of the phage MS-2 and of the gene cII of bacteriophage lambda. The Shine-Dalgarno sequence may be directly followed by DNA encoding SSTR- protein and result in the expression of the mature SSTR- protein.

Moreover, suitable expression vectors can include an appropriate marker that allows the screening of the transformed host cells. The transformation of the selected host is carried out using any one of the various techniques well known to the expert in the art and described in Sambrook et al., *supra.*

An origin of replication may also be provided either by construction of the vector to include an exogenous origin or may be provided by the host cell chromosomal replication mechanism. If the vector is integrated into the host cell chromosome, the latter may be sufficient. Alternatively, rather than using vectors which contain viral origins of replication, one skilled in the art can transform mammalian cells by the method of co-transformation with a selectable marker and SSTR- protein encoding DNA. An example of a suitable marker is dihydrofolate reductase or thymidine kinase (see, U.S. Pat. No. 4,399,216).

Nucleotide sequences encoding reporter protein fusions, such as SSTR2- proteins, may be recombined with vector DNA in accordance with conventional techniques, including blunt-ended or staggered-ended termini for ligation, restriction enzyme digestion to provide appropriate termini, filling in of cohesive ends as appropriate, alkaline phosphatase treatment to avoid undesirable joining, and ligation with appropriate ligases. Techniques for such manipulation are disclosed by Sambrook et al., supra and are well known in the art. Methods for construction of mammalian expression vectors are disclosed in, for example, Okayama et al., MOL. CELL. BIOL., 3:280, (1983); Cosman et al., MOL. IMMUNOL., 23:935, (1986); and, Cosman et al., NATURE, 312: 768, (1984).

The transgene construct preferably includes a leader sequence downstream from the promoter. The leader sequence is a nucleic acid sequence that encodes a protein secretory signal, and, when operably linked to a downstream nucleic acid molecule encoding the ATIII- protein of the invention, and directs ATIII-secretion. The leader sequence may be obtained from the same gene as the promoter used to direct transcription of the nucleic acid molecule encoding ATIII (for example, a gene that encodes a milk-specific protein). Alternatively, a leader sequence encoding the native human ATIII protein secretory signal (amino acids 1-19 of Genbank Accession No. V01514) may be employed.

### Therapeutic Uses.

The combination herein is preferably employed for *in vitro* use in treating these tissue cultures. The combination, however, is also be effective for *in vivo* applications. Depending on the intended mode of administration *in vivo* the compositions used may be in the dosage form of solid, semi-solid or liquid such as, e.g., tablets, pills, powders, capsules, gels, ointments, liquids, suspensions, or the like. Preferably the compositions are administered in unit dosage forms suitable for single administration of precise dosage amounts. The compositions may also include, depending on the formulation desired, pharmaceutically acceptable carriers or diluents, which are defined as aqueous-based vehicles commonly used to formulate pharmaceutical compositions for animal or human administration. The diluent is selected so as not to affect the biological activity of the human alpha-fetoprotein. Examples of such diluents are distilled water, physiological saline, Ringer's solution, dextrose solution, and Hank's solution. The same diluents may be used to reconstitute lyophilized human alpha-fetoprotein. In addition, the pharmaceutical composition may also include other medicinal agents, pharmaceutical agents, carriers, adjuvants, nontoxic, non-therapeutic, non-immunogenic stabilizers, etc. Effective amounts of such diluent or carrier will be amounts which are effective to obtain a pharmaceutically acceptable formulation in terms of solubility of components, biological activity, etc.

The compositions herein may be administered to human patients via aerosolized, nebullized, pulmonary, parenteral or oral administrations and otherwise systemic forms for acute lung injuries resulting from smoke inhalation and/or bum damage.

### Bacterial Expression.

Useful expression vectors for bacterial use are constructed by inserting a structural DNA sequence encoding a desired protein together with suitable translation initiation and termination signals in operable reading phase with a functional promoter. The vector will comprise one or more phenotypic selectable markers and an origin of replication to ensure maintenance of the vector and, if desirable, to provide amplification within the host. Suitable prokaryotic hosts for transformation include E. *coli., Bacillus subtilis, Salmonella typhimurium* and various species within the genera *Pseudomonas, Streptomyces,* and *Staphylococcus,* although others may, also be employed as a matter of choice. In a preferred embodiment, the prokaryotic host is *E*. *coli.*

Bacterial vectors may be, for example, bacteriophage-, plasmid- or cosmid-based. These vectors can comprise a selectable marker and bacterial origin of replication derived from commercially available plasmids typically containing elements of the well known cloning vector pBR322 (ATCC 37017). Such commercial vectors include, for example, GEM 1 (Promega Biotec, Madison, Wis., USA), pBs, phagescript, PsiX174, pBluescript SK, pBs KS, pNH8a, pNH16a, pNH18a, pNH46a (Stratagene); pTrc99A, pKK223-3, pKK233-3, pKK232-8, pDR540, and pRIT5 (Pharmacia). A preferred vector according to the invention is THE Pt7I expression vector.

These "backbone" sections are combined with an appropriate promoter and the structural sequence to be expressed. Bacterial promoters include lac, T3, T7, lambda PR or PL, trp, and ara. T7 is a preferred bacterial promoter.

Following transformation of a suitable host strain and growth of the host strain to an appropriate cell density, the selected promoter is de-repressed/induced by appropriate means (e.g., temperature shift or chemical induction) and cells are cultured for an additional period. Cells are typically harvested by centrifugation, disrupted by physical or chemical means, and the resulting crude extract retained for further purification.

### Eukaryotic Expression

Various mammalian cell culture systems can also be employed to express recombinant protein. Examples of mammalian expression systems include selected mouse L cells, such as thymidine kinase-negative (TK) and adenine phosphoribosul transferase-negative (APRT) cells. Other examples include the COS-7 lines of monkey kidney fibroblasts, described by Gluzman, Cell 23:175 (1981), and other cell lines capable of expressing a compatible vector, for example, the C127, 3T3, CHO, HeLa and BHK cell lines. In particular, as regards yeasts, there may be mentioned yeasts of the genus *Saccharomyces, Kluyveromyces, Pichia, Schwanniomyces,* or *Hansenula.* Among the fungi capable of being used in the present invention, there may be mentioned more particularly *Aspergillus* ssp, or *Trichoderma* ssp.

Mammalian expression vectors will comprise an origin of replication, a suitable promoter and enhancer, and also any necessary ribosome binding sites, polyadenylation site, splice donor and acceptor sites, transcriptional termination sequences, and 5' flanking non-transcribed sequences. DNA sequences derived from the SV40 viral genome, for example, SV40 origin, early promoter, enhancer, splice, and polyadenylation sites may be used to provide the required non-transcribed genetic elements.

Mammalian promoters include beta-casein, beta-lactoglobulin, whey acid promoter others include: HSV thymidine kinase, early and late SV40, LTRs from retrovirus, and mouse metallothionein-1. Exemplary mammalian vectors include pWLneo, pSV2cat, pOG44, pXT1, pSG (Stratagene) pSVK3, pBPV, pMSG, and pSVL (Pharmacia). In a preferred embodiment, the mammalian expression vector is pUCIG-MET. Selectable markers include CAT (chloramphenicol transferase).

The nucleotide sequences which can be used within the framework of the present invention can be prepared in various ways. Generally, they are obtained by assembling, in reading phase, the sequences encoding each of the functional parts of the polypeptide. The latter may be isolated by the techniques of persons skilled in the art, and for example directly from cellular messenger RNAs (mRNAs), or by recloning from a complementary DNA (cDNA) library, or alternatively they may be completely synthetic nucleotide sequences. It is understood, furthermore, that the nucleotide sequences may also be subsequently modified, for example by the techniques of genetic engineering, in order to obtain derivatives or variants of the said sequences.

### Therapeutic Compositions.

The proteins of the present invention can be formulated according to known methods to prepare pharmaceutically useful compositions, whereby the inventive molecules, or their functional derivatives, are combined in admixture with a pharmaceutically acceptable carrier vehicle. Suitable vehicles and their formulation, inclusive of other human proteins, e.g., human serum albumin, are described, for example, in order to form a pharmaceutically acceptable composition suitable for effective administration, such compositions will contain an effective amount of one or more of the proteins of the present invention, together with a suitable amount of carrier vehicle.

Pharmaceutical compositions for use in accordance with the present invention may be formulated in conventional manner using one or more physiologically acceptable carriers or excipients. Thus, the bi-functional molecules and their physiologically acceptable salts and solvate may be formulated for administration by inhalation or insufflation (either through the mouth or the nose) or oral, buccal, parenteral or rectal administration.

For oral administration, the pharmaceutical compositions may take the form of, for example, tablets or capsules prepared by conventional means with pharmaceutically acceptable excipients such as binding agents (e.g., pregelatinised maize starch, polyvinylpyrrolidone or hydroxypropyl methylcellulose); fillers (e.g., lactose, microcrystalline cellulose or calcium hydrogen phosphate); lubricants (e.g., magnesium stearate, talc or silica); disintegrants (e.g., potato starch or sodium starch glycolate); or wetting agents (e.g., sodium lauryl sulphate). The tablets may be coated by methods well known in the art, Liquid preparations for oral administration may take the form of, for example, solutions, syrups or suspensions, or they maybe presented as a dry product for constitution with water or other suitable vehicle before use. Such liquid preparations may be prepared by conventional means with pharmaceutically acceptable additives such as suspending agents (e.g., sorbitol syrup, cellulose derivatives or hydrogenated edible fats); emulsifying agents (e.g., lecithin or acacia); non-aqueous vehicles (e.g., almond oil, oily esters, ethyl alcohol or fractionated vegetable oils); and preservatives (e.g., methyl or propyl-p-hydroxybenzoates or sorbic acid). The preparations may also contain buffer salts, flavoring, coloring and sweetening agents as appropriate.

Preparations for oral administration may be suitably formulated to give controlled release of the active compound. For buccal administration the composition may take the form of tablets or lozenges formulated in conventional manner.

For administration by inhalation, the bi-functional molecules for use according to the present invention are conveniently delivered in the form of an aerosol spray presentation from pressurized packs or a nebullizer, with the use of a suitable propellant, e.g., dichlorodifluoromethane, trichlorofluoromethane, dichlorotetrafluoroethan- e, carbon dioxide or other suitable gas. In the case of a pressurized aerosol the dosage unit may be determined by providing a valve to deliver a metered amount. Capsules and cartridges of, e.g. gelatin for use in an inhaler or insufflator may be formulated containing a powder mix of the compound and a suitable powder base such as lactose or starch.

The multiple proteins of the composition of the invention may be formulated for parenteral administration by injection, e.g., by bolus injection or continuous infusion. Formulations for injection may be presented in unit dosage form, e.g., in ampules or in multi-dose containers, with an added preservative. The compositions may take such forms as suspensions, solutions or emulsions in oily or aqueous vehicles, and may contain formulatory agents such as suspending, stabilizing and/or dispersing agents. Alternatively, the active ingredient may be in powder form for constitution with a suitable vehicle, e.g., sterile pyrogen-free water, before use.

The compounds may also be formulated in rectal compositions such as suppositories or retention enemas, e.g., containing conventional suppository bases such as cocoa butter or other glycerides.

In addition to the formulations described previously, the bi-functional molecules may also be formulated as a depot preparation. Such long acting formulations may be administered by implantation (for example subcutaneously or intramuscularly) or by intramuscular injection. Thus, for example, the compounds may be formulated with suitable polymeric or hydrophobic materials (for example as an emulsion in an acceptable oil) or ion exchange resins, or as sparingly soluble derivatives, for example, as a sparingly soluble salt.

The compositions may, if desired, be presented in a pack or dispenser device which may contain one or more unit dosage forms containing the active ingredient. The pack may for example comprise metal or plastic foil, such as a blister pack. The pack or dispenser device may be accompanied by instructions for administration.

### Literature Cited and Incorporated by Reference:

1. Abubakar K, Schmidt B, Monkman S, et al., Heparin Improves Gas Exchange During Ex-Perimental Acute Lung Injury In Newborn Piglets. AM J RESPIR CRIT CARE MED 1998; 158:1620-1625.
2. Arocas V, Turk B, Bock SC, Olson ST, Bjork I (2000), The Region of Antithrombin Interacting with Full-Length Heparin Chains Outside the High-Affinity Pentasaccharide Sequence Extends to Lys 136 But Not to Lys 139. BIOCHEMISTRY 39:8512-8.
3. Desai U, Swanson R, Bock SC, Bjork I, Olson ST (2000), Role OfArginine 129 In Heparin Binding And Activation Of Antithrombin. J. BIOL. CHEM. 275:18976-84.
4. Edmunds, T., S. M. Van Patten, J. Pollock, E. Hanson, R. Bernasconi, E. Higgins, P. Manavalan, C. Ziomek, H. Meade, J. M. McPherson, and E. S. Cole et al., 1998, Transgenically Produced Human Antithrombin: Structural And Functional Comparison To Human Plasma-Derived Antithrombin. BLOOD 91(12):4561-71.
5. Gross, T. J., R. H. Simon, and R. G. Sitrin. 1992. Tissue Factor Procoagulant Expression By Rat Alveolar Epithelial Cells. AM J RESPIR CELL MOL BIOL 6(4):397-403.
6. Herndon, D. N., D. L. Traber, G. D. Niehaus, H. A. Linares, and L. D. Traber. 1984. The Pathophysiology Of Smoke Inhalation Injury In A Sheep Model, J TRAUMA 24(12):1044-51.
7. Horie S, Ichii H, Kazama M. et al., Heparin-Likeglycosaminoglycan Is A Receptor For Anti-Thrombin III-Dependent But Not Thrombin-Dependent Prostacyclin Production In Humanendothelial Cells. THROMB RES 1990; 59:899 -904
8. Idell, S., A. P. Mazar, P. Bitterman, S. Mohla, and A. L. Harabin. 2001. Fibrin Turnover In Lung Inflammation And Neoplasia. AM J RESPIR CRIT CARE MED 163(2):578-84,
9. Isobe, H., K. Okajima, M. Uchiba, N. Harada, and H. Okabe. 2002. Antithrombin Prevents Endotoxin-Induced Hypotension By Inhibiting The Induction Of Nitric Oxide Synthase In Rats. BLOOD 99(5):1638-45.
10. Jesty J, Lorenz A, Rodriguez J, et al., Initiation Of Tissue Factor Pathway Of Coagulation In The Presence Of Heparin: Control By Anti-Thrombin III And Tissue Factor Pathway Inhibitor. BLOOD 1996; 15:2301-2307
11. Kaneider, N. C., P. Egger, S. Dunzendorfer, and C. J. Wiedermann, 2001. Syndecan-4 As Antithrombin Receptor Of Human Neutrophils. BIOCHEM BIOPHYS RES COMMUN 287(1):42-6.
12. Kaneider, N. C., C. M. Reinisch, S. Dunzendorfer, J. Romisch, C. J. Wiedermann, and C. J. Wiederman. 2002. Syndecan-4 Mediates Antithrombin-Induced Chemotaxis Of Human Peripheral Blood Lymphocytes And Monocytes. J CELL SCI 115(Pt 1):227-36.
13. Kimura, R., L. D. Traber, D. N. Herndon, G. D. Neuhaus, and D. L. Traber. 1988. Treatment Of Smoke-Induced Pulmonary Injury With Nebulized Dimethylsulfoxide. CIRC SHOCK 25(4):333-41.
14. Kimura R, Traber DL, Herndon DN, et al: Increasing Duration Of Sinoke Exposure Induces More Severe Lung Injury In Sheep. J APPL PHYSIOL 1988; 64:1107-1113
15. Kowal-Vern, A., V. McGill, J. M. Walenga, and R. L. Gamelli. 2000. Antithrombin(H) Concentrate Infusions Are Safe And Effective In Patients With Thermal Injuries. J BURN CARE REHABIL 21 (2):115-27.
16. Kowal-Vern, A., R. L. Gamelli, J. M. Walenga, D. Hoppensteadt, M. Sharp-Pucci, and H. R. Schumacher. 1992. The Effect Of Burn Wound Size On Hemostasis: A Correlation Of The Hemostatic Changes To The Clinical State. J TRAUMA 33(1):50-6; discussion 56-7.
17. McKeage, K., and G L. Plosker. 2001. HEPARIN. DRUGS 61 (4):515-22; discussion 523-4.
18. Minnema, M. C., A. C. Chang, P. M. Jansen, Y T. Lubbers, B. M. Pratt, B. G. Whittaker, F. B. Taylor, C. E. Hack, and B. Friedman. 2000. Recombinant Human Antithrombin III Improves Survival And Attenuates Inflammatory Responses In Baboons Lethally Challenged With Escherichia Coli. BLOOD 95(4):1117-23.
19. Mizutani, A., K. Okajima, M. Uchiba, H. Isobe, N. Harada, S. Mizutani, and T. Noguchi. 2003. Antithrombin Reduces Ischemia/Reperfusion-Induced Renal Injury In Rats By Inhibiting Leukocyte Activation Through Promotion Of Prostacyclin Production. BLOOD 101(8):3029-36.
20. Murakami, K., L. J. Bjertnaes, F. C. Schmalstieg, R. McGuire, R. A. Cox, H. K. Hawkins, D. N. Herndon, L. D. Traber, and D. L. Traber. 2002. A Novel Animal Model Of Sepsis After-Acute Lung Injury In Sheep. CRIT CARE MED 30(9):2083-90.
21. Murakami, K., R. McGuire, R. A. Cox, J. M. Jodoin, L. J. Bjertnaes, J. Katahira, L. D. Traber, F. C. Schmalstieg, H. K. Hawkins, D. N. Herndon, and D. L. Traber. 2002. Heparin Nebulization Attenuates Acute Lung Injury In Sepsis Following Smoke Inhalation In Sheep. SHOCK 18(3):236-41.
22. Murakami, K., R. McGuire, R. A. Cox, J. M. Jodoin, F. C. Schmalstieg, L. D. Traber, H. K. Hawkins, D. N. Herndon, and D. L. Traber. 2003. Recombinant αntithrombin attenuates pulmonary inflammation following smoke inhalation and pneumonia in sheep. CRIT CARE MED 31(2):577-83.
23. Oelschlager, C., J. Romisch, A. Staubitz, H. Stauss, B. Leithauser, H. Tillmanns, and H. Holschermann. 2002. Antithrombin III Inhibits Nuclear Factor kappaB Activation In Human Monocytes And Vascular Endothelial Cells. BLOOD 99(11):4015-20.
24. Olson ST, Bjork I, Bock SC (2002) Identification of Critical Molecular Interactions which Mediate Heparin Activation of Antithrombin: Implications for the Design of Improved Heparin Anticoagulants. TRENDS IN CARDIOVASCULAR MEDICINE.
25. Opal, S. M. 2000. Therapeutic Rationale For Antithrombin III In Sepsis. Crit Care Med 28(9 Suppl):S34-7.
26. Salvatierra A, Guerrero R, Rodriguez M, et al., Antithrombin III Prevents Early Pulmonary Dysfunction After Lung Transplantation In The Dog, CIRCULATION 2001; 104:2975-2980
27. Schmalstieg, F. C., J. Chow, C. Savage, H. E. Rudloff, K. H. Palkowetz, and J. B. Zwischenberger. 2001. Interleukin-8, Aquaporin-1, And Inducible Nitric Oxide Synthase In Smoke And Burn Injured Sheep Treated With Percutaneous Carbon Dioxide Removal. ASAIO J 47(4):365-71
28. Seeger, W., C. Grube, and A. Gunther. 1993. Proteolytic Cleavage Of Fibrinogen: Amplification Of Its Surfactant Inhibitory Capacity. AM J RESPIR CELL MOL BIOL 9(3):239-47.
29. Sitrin, R. G., P. G. Brubaker, J. E. Shellito, and H. B. Kaltreider. 1986. The Distribution Of Procoagulant And Plasminogen Activator Activities Among Density Fractions Of Normal Rabbit Alveolar Macrophages. AM REV RESPIR DIS 133(3):468-72.
30. Soejima, K., L. D. Traber, F. C. Schmalstieg, H. Hawkins, J. M. Jodoin, C. Szabo, E. Szabo, L. Varig, A. Salzman, and D. L. Traber. 2001. Role Of Nitric Oxide In Vascular Permeability After Combined Burns And Smoke Inhalation Injury. AM J RESPIR CRIT CARE MED 163(3 Pt 1):745-52.
31. Tasaki, O., D. W. Mozingo, M. A. Dubick, C. W. Goodwin, L. D. Yantis, and B. A. Pruitt, Jr. 2002. Effects Of Heparin And Lisofylline On Pulmonary Function After Smoke Inhalation Injury In An Ovine Model. CRIT CARE MED 30(3):637-43.
32. Uchiba, M., K. Okajima, K. Murakami, H. Okabe, and K. Takatsuki. 1995. Effects Of Antithrombin III (AT III) And Trp49-Modified AT III On Plasma Level Of 6-Keto-PGFI Alpha In Rats. Thromb Res 80(3):201-8.
33. Uchida M, Okajima K, Murakami K: Effects Of Various Doses OfAntithrombin III And Endotoxin-Induced Endothelial Cell Injury And Coagulation Abnormalities In Rats. THROMB RES1998; 89:233-241.
34. von Bethmann, A. N., F. Brasch, R. Nusing, K. Vogt, H. D. Volk, K. M. Muller, A. Wendel, and S. Uhlig. 1998. Hyperventilation Induces Release Of Cytokines From Perfused Mouse Lung. AM J RESPIR CRIT CARE MED 157(1):263-72.
35. Vervloet, M. G., L. G. Thijs, and C. E. Hack. 1998. Derangements Of Coagulation And Fibrinolysis In Critically Ill Patients With Sepsis And Septic Shock. SEMIN THROMB HEMOST 24(1):33-44.
36. Wiedermann Ch, J., and J. Romisch. 2002. The Anti-Inflammatory Actions Of Antithrombin-A Review. ACTA MED AUSTRIACA 29(3):89-92.
37. Yamanuchi T, Umeda F, Inoguchi I, et al., Antithrombin III Stimulates Prostacyclin Production By Cultured Aortic Endothelial Cells. BIOCHEM BIOPHYS RES COMMUN 1989;163:1404 -1411 S335 Crit Care Med 2003 Vol. 31, No. 4 (Suppl.),

## Claims

1. Use of antithrombin III and heparin in the preparation of a medicament for use in a method of treating acute lung injury in a subject, the method comprising:
administering a therapeutically effective amount of antithrombin III and heparin such that the lung injury is treated.

2. The use of claim 1, wherein the antithrombin III is administered by inhalation.

3. The use of claim 1, wherein the antithrombin III is administered intravenously.

4. The use of claim 2 or 3, wherein the heparin is administered by inhalation.

5. The use of claim 2 or 3, wherein the heparin is administered intravenously.

6. The use of any one of claims 1-5, wherein the antithrombin III and heparin are administered concurrently.

7. The use of any one of claims 1-6, wherein the antithrombin III is administered at a dose of about 10-300 U/kg of body weight, preferably at a dose of about 25-125 U/kg of body weight.

8. The use of any one of claims 1-6, wherein the heparin is administered at a dose of about 10-300 U/kg of body weight, preferably at a dose of about 25-125 U/kg of body weight.

9. The use of any one of claims 1-6, wherein the antithrombin III is selected from plasma derived antithrombin III, recombinantly produced antithrombin III, and transgenically produced antithrombin III.

10. The use of any one of claims 1-6, wherein the heparin is plasma derived heparin.

11. The use of any one of claims 1-6, wherein the heparin is administered using an ultrasonic nebulizer, a dry powder inhalation system, or a jet aerosol.

12. The use of any one of claims 1-6, wherein said method of treatment is effective in increasing the pulmonary gas exchange fraction; or in decreasing vascular permeability.

13. The use of claim 1, wherein the lung injury was caused by smoke inhalation; was caused by burn damage to the lungs; is septic acute lung injury; is acute respiratory distress syndrome (ARDS); is in response to exposure to an external agent, preferably the external agent being selected from *Pseudomonas pneumonia,* asbestos, or a viral agent; comprises lung neoplasia; comprises pleural neoplasia; comprises interstitial lung disease; or comprises organizing pleuritis.

14. Use of antithrombin III in the preparation of a medicament for use in a method for treating acute lung injury in a subject by administering a therapeutically effective amount of aerosolized heparin and antithrombin III such that the lung injury is treated.

15. Use of heparin in the preparation of a medicament for use in a method for treating acute lung injury in a subject by administering a therapeutically effective amount of aerosolized heparin and antithrombin III such that the lung injury is treated.
